# EUROPEAN PATENT SPECIFICATION

(11) **EP 1 307 467 B1**
(45) Date of publication and mention of the grant of the patent: **20.07.2005**
(21) Application number: 01954187.9
(22) Date of filing: 07.08.2001
(51) Int. Cl.: C07H 1/06

(54) **METHOD OF SEPARATING NUCLEOSIDE PHOSPHATES**
VERFAHREN ZUR TRENNUNG VON NUKLEOSIDPHOSPHATEN
PROCEDE DE SEPARATION DE NUCLEOSIDES-PHOSPHATES

(30) Priority: 08.08.2000 GB 0019322
(43) Date of publication of application: 07.05.2003
(73) Proprietor: ISIS INNOVATION LIMITED, Summertown, Oxford OX2 7SG (GB)
(72) Inventor: O'HARE, Dermot, Michael The University of Oxford, Oxford OX1 3QR (GB); LOTSCH, Bettina, 81627 Munich (DE)
(74) Representative: Wilkinson, Stephen John
(86) International application number: PCT/GB2001/003548
(87) International publication number: WO 2002/012255

(56) References cited:
- WO-A-99/24139
- CHOY: "Intercalative nanohybrids of nucleoside monophosphate and DNA in layered metal hydroxide" J. AM. CHEM. SOC., vol. 121, 1999, pages 1399-1400, XP002184245 cited in the application & EP 0 987 328 A (CHOY) 22 March 2000 (2000-03-22)
- MEYN M ET AL: "ANION-EXHANGE REACTIONS OF LAYERED DOUBLE HYDROXIDES" INORGANIC CHEMISTRY, AMERICAN CHEMICAL SOCIETY, EASTON, US, vol. 29, no. 26, 26 December 1990 (1990-12-26), pages S201-S207, XP000567549 ISSN: 0020-1669
- BORJA, MARLON ET AL: "Fatty acids in layered metal hydroxides: membrane-like structure and dynamics" J. PHYS. CHEM. (1992), 96(13), 5434-44 , 1992, XP002184246

## Description

The present invention relates to a method of separating nucleoside phosphates. More particularly, it relates to a method by which a nucleoside phosphate can be separated from a mixture containing it and, particularly, from a mixture containing it and one or more other nucleoside phosphates using a layered double hydroxide.

Processes for separating compounds from mixtures are of great importance, both in the laboratory and on an industrial scale. The purity of chemical compounds is dictated to a large extent by the purification step in which a compound is separated from other products of the chemical reaction by which it is produced. No simple process is known by which a nucleoside phosphate can be separated from a mixture, and particularly from a mixture of nucleoside phosphates containing it. The benefits of such a procedure would be welcome to industries producing or using such compounds.

Layered double hydroxides (LDHs) are a class of compounds which comprise two metal cations and have a layered structure. A brief review of LDHs is provided in Chemistry in Britain, September 1997, pages 59 to 62. The hydrotalcites, perhaps the most well-known examples of LDHs, have been studied for many years.

LDHs can be represented by the general formula [M^{II}₍₁₋ₓ₎M^{III}ₓ(OH)₂]^{x+}A^{z-}_{x/z}.yH₂O or [M^{I}₍₁₋ₓ₎M^{III}ₓ(OH)₂]ⁿ⁺A^{z-}_{n/z}.yH₂O, where M^{I}, M^{II} and M^{III} are mono, di- and trivalent metal cations respectively, that occupy octahedral positions in hydroxide layers, A^{z-} is an interlayer charge-compensating anion where z is an integer, such as CO₃²⁻, NO₃⁻ or Cl⁻, n=2x-1, x is a number less than 1 and y is 0 or a number greater than 0. A large number of LDHs with a wide variety of M^{II}-M^{III} cation pairs (e.g., Ca-Al) as well as the M^{I}-M^{III} cation pair (Li-Al) with different anions in the interlayer space have been reported and studied.

It is known that certain organic species may be intercalated into the layers in some LDHs and into clays. For example, Ogawa et al., in Chemistry Letters, 1992, no. 3, p.365-368, describe the intercalation of maleic and methylmaleic acids into the clay montmorillonite in a solid state reaction. The geometrical isomers of the acids, fumaric and methylfumaric acids, were not intercalated in the solid state reaction. However, when an ethanolic solution of the two isomers was used, the montmorillonite showed no selectivity and both isomers were intercalated.

The structure of the layered materials [LiAl₂(OH)₆]X, where X is Cl, Br or NO₃, and their hydrates has been described by Besserguenev et al., in Chem. Mater, 1997, no. 9, p.241-247. The materials can be produced by the reaction of gibbsite [γ-Al(OH)₃] or other forms of Al(OH)₃, such as bayerite, nordstrandite or doyleite, with lithium salts of formula LiX. The materials can also be formed in other ways, such as by direct precipitation (see, for example, Sema et al., Clays & Clay Minerals, (1997), 25,384). The structure of the LiAl₂(OH)₆⁺ layers in the compounds is unusual amongst LDHs since it is based on an ordered arrangement of metal cations within the layers.

The synthesis of LiAl₂(OH)₆⁺ compounds is described in US 4,348,295 and US 4,348,297. The use of the materials for separating hydrocarbons and for gas chromatograph columns is taught in US 4,430,097 and US 4,321,065, respectively. In both of these latter two documents, the technology described does not involve intercalation chemistry but surface interactions with the stationary phase i.e., liquid-solid or gas-solid interactions.

Intercalates of compounds of formula LiOH.2Al(OH)₃ are described in US 4,727,167 and US 4,812,245. Both documents relate to uses of the intercalates as additives to organic materials such as mineral oils.

A few other LDHs having cation ordering are known. The layered double hydroxide [Ca₂Al(OH)₆]₂⁺SO₄²⁻ is an example.

LDHs exhibit a wide range of anion-exchange reactions with guests such as organic carboxylates, sulfonates and a range of anionic metal complexes. These materials are of significant technological importance in diverse areas such as catalysis, optics, medical science and separation science.

The application of LDHs in separation science has until recently been largely restricted to their role as fast, efficient, high capacity ion-exchange materials. The major application being the removal of organic and inorganic anions from aqueous streams. According to WO 99/24139 a compound comprising at least two negatively charged groups connected by a linker group can be separated from a mixture containing it by selectively intercalating it into an LDH. For example, it was disclosed therein that when [LiAl₂(OH)₆]Cl.H₂O is treated with an equimolar mixture of the disodium salts of either the 1,2-, 1,3- or 1,4-dibenzoic acids then the only crystalline phase observed is formed by preferential and exclusive intercalation of the 1,4-dibenzoate anions.

Choy et al., J.Am.Chem.Soc. 1999, 121, 1399-1400, and EP-A-0987328 have reported that the nucleoside monophosphates such as adenosine-5'monophosphate (AMP), guanosine-5'-monophosphate (GMP) and cytidine-5'-monophosphate (CMP) can be ion-exchange intercalated in the layered double hydroxide Mg_{0.68}Al_{0.32}(OH)₂](NO₃)_{0.32}.1.2H₂O.

The present invention provides a method of separating a first nucleoside phosphate from a solution containing it and at least a second nucleoside phosphate which method comprises treating the solution with a layered double hydroxide whereby the first nucleoside phosphate is preferentially intercalated into the layered double hydroxide and then recovering the intercalated nucleoside phosphate from the layered double hydroxide.

By the method of the present invention it is possible to separate one nucleoside phosphate from a solution containing it and at least one other nucleoside phosphate. The term "nucleoside phosphate" as used herein means any nucleoside in which the sugar component, i.e., the deoxyribose or ribose molecule which is attached to the purine or pyrimidine base is attached, typically at its 3' or 5'-position and preferably at its 5'-position, to a phosphate group, such as a monophosphate, a diphosphate or a triphosphate group. Thus, it is believed that the method of the present invention provides a way of separating any nucleotide from a mixture containing two or more nucleotides. More particularly, the method has utility in separating a nucleoside monophosphate from a mixture of two or more nucleoside monophosphates, or separating a nucleoside diphosphate from a mixture of two or more nucleoside diphosphates, or separating a nucleotide triphosphate from a mixture of two or more nucleoside triphosphates. For instance, cytidine-5'-monophosphate can be separated from a dissolved mixture containing it and adenosine-5'-monophosphate or from a mixture containing it and guanosine-5'-monophosphate. Similarly, guanosine-5'-monophosphate can be separated, according to the present invention, from a dissolved mixture with adenosine-5'-monophosphate.

The layered double hydroxide that can be used in the method of the invention comprises two metal cations and hydroxide ions and has a layered structure. Between the layers, the LDH contains an intercalated anionic group which is one that can be displaced by the nucleoside phosphate, or at least part thereof. A first class of LDH compounds having use in the present invention has the general formulae [M^{I}₍₁₋ₓ₎M^{III}ₓ(OH)₂]ⁿ⁺A^{z-}_{n/z} in which M^{I} is a monovalent metal cation, M^{III} is a trivalent metal cation, A is a displaceable anion, z is an integer and n = 2x-1, where x is a number less than 1. These compounds are optionally, but preferably, hydrated. The amount of water of hydration may be a stoichiometric or non-stoichiometric amount. Of this class of LDH compounds, those wherein M^{I} is a lithium ion and M^{III} is an aluminium ion are particularly preferred such that the layers in the compound comprise [LiAl₂(OH)₆]⁺. The cations in such a layer have an ordered arrangement, i.e., the LDH has cation ordering. The ordered (i.e., non-random) arrangement of cations is believed to be beneficial and enhance the selectivity of the method of the invention.

In the above formula given for the first class LDH compounds useful in the present invention the anion A is one that can be displaced from its location between the metal cation containing layers by a nucleoside phosphate, for example it may be an anion selected from hydroxide, a halide (i.e., fluoride, chloride, bromide and iodide), sulphate or nitrate ions. Preferably, the anion A is chloride or nitrate.

A second class of LDH compounds that can be used in the present invention to separate nucleoside phosphates has the general formula

[M^{II} ₍₁₋ₓ₎M^{III} ₓ(OH)₂]^{x+}A^{z-} _{x/z}

wherein M" is a divalent metal cation, for example, Ca²⁺, Mg²⁺, Zn²⁺ and Ni²⁺ ions, M^{III} is a trivalent metal cation, for example, Al³⁺, Cr³⁺ and Fe³⁺ ions, A is a displaceable anionic group such as one disclosed above in connection with the described first class of LDH compounds z is an integer and x is a number less than 1. Such compounds are optionally, but preferably, hydrated. The water of hydration may be present in a stoichiometric amount or a non-stoichiometric amount.

Examples of LDH compounds that are preferred for use in the present invention include LiAl₂(OH)₆Cl.H₂O, Mg₂Al(OH)₆NO₃.2H₂O, Zn₂Cr(OH)₆NO₃.2H₂O and Ni₂Al(OH)₆Cl.2H₂O.

The method of the present invention involves the treatment of a solution containing the nucleoside phosphates, or metal salts thereof, to be separated with the LDH compound. The solution containing the dissolved nucleoside phosphates may be prepared using any solvent in which the phosphates dissolve and which does not prejudice the intercalation reaction. Typically the solvent will be a polar solvent, for example water, an alcohol, THF or CH₃CN. The use of an aqueous solution of nucleoside phosphates and, thus, the use of an aqueous reaction medium is especially preferred in view of the obvious advantages associated with this, for example, ease of handling, costs and the good solvent properties of water. The solution treated with the LDH should not contain any chemical species that will intercalate into the LDH in preference to the nucleoside phosphate. The temperature at which the treatment is carried out may also affect the selectivity of the process. For instance, when using the LDH LiAl₂(OH)₆Cl.H₂O to treat an aqueous solution of Na₂GMP and Na₂AMP, the sodium salts of guanosine-5'-monophosphate and adenoside-5'-monophosphate, respectively, the selectivity ratio (Na₂GMP:Na₂AMP) was found to be about 31:69 at a treatment temperature of 30°C but was found to be 86:14 at a treatment temperature of 100°C. These results show that different compounds are preferentially intercalated from the same mixture at different temperatures. Preferably, the method is carried out at a temperature within the range of from 40 to 100°C. The fact that the temperature of the reaction mixture used in the method can influence the selectivity of the method means that the method can be employed to target a particular compound for separation from its mixture with another compound. It also means that the same mixture of compounds can be used for the separation of different compounds from the mixture simply by varying the reaction temperature. Suitable reaction temperatures can be readily determined by routine experimentation in each case. Thus, the method of the invention may involve selection of the reaction temperature used so as to optimise the intercalation of the desired compound into the LDH.

In order to effect intercalation, particulate LDH can be mixed into an aqueous solution containing a mixture of nucleoside phosphates. Alternatively, an aqueous solution containing the mixture of nucleoside phosphates may be added to an aqueous suspension of the LDH. Preferably, an aqueous suspension of the LDH is treated by ultrasonication for a period of time of from 1 to 60 minutes, preferably from 30 to 45 minutes, prior to mixing this with the solution containing the nucleoside phosphates since this prior treatment enhances the extent of the intercalation reaction. Treatment by ultrasonication of the LDH is believed to reduce the particle size of the LDH particles and, therefore, increase the surface area of the LDH which is accessible to intercalation by the nucleoside phosphates.

Typically, the reaction is carried out by stirring the aqueous mixture of the reactants for a period of time within the range of from 15 minutes to 24 hours. As revealed by time-resolved, *in situ*, energy dispersive X-ray diffraction studies on the intercalation of AMP and of CMP into LiAl₂(OH)₆Cl·H₂O, the reaction may be complete within about 25 minutes for AMP and within about 120 minutes for CMP, if the guest solution is added dropwise. The reaction time is likely to be even faster if a batchwise method is used. Extension of the reaction time results in slightly enhanced crystallinity of the product, which may be ascribed to the phenomenon of crystal growth by ageing.

Intercalation has been found to be complete at molar guest: host ratios in the range of from 0.5:1 to 3:1, corresponding to stoichiometric ratios in the range of from 1:1 to 6:1. Though not complete, intercalation of AMP into LiAl₂(OH)₆Cl·H₂O was also observed at substoichiometric AMP:LDH ratios (corresponding to a molar ratio of AMP:LDH of 0.25:1), indicating that replacement of the chloride ion in the interlayer spaces by AMP is favoured at high temperatures (e.g. 80°C) yet no staging was observed. In some experiments, guest : host ratios of ≥ 3, (or significantly decreased volumes of the reaction mixture), seemed to impede intercalation. This might be ascribed to an aggregation of the nucleotide molecules occurring in solution or to blocking effects of adsorbed guest molecules at the edges of the LDH particles which prevent further diffusion of the guest molecules into the interlayer spaces in the LDH. The nucleoside phosphates in the solution treated with the LDH according to the invention will, preferably, be in a molar excess with respect to the LDH. More preferably, the molar ratio of nucleoside phosphate : LDH will be 2:1 since this guest: host ratio is found to be optimal for the intercalation reaction. In the case of the nucleoside monophosphates the reaction solution typically has a concentration of 0.07M in the nucleotide and 0.035M in the LDH.

After the intercalation reaction the particles of the LDH containing intercalated nucleoside phosphate are separated from the aqueous medium, typically by filtration. Alternatively, the aqueous mixture of nucleoside phosphates may be passed through the LDH. Conventional chromatographical and filtration techniques can be used for this purpose. For example, the LDH can be provided as a chromatographic column and the solution of nucleoside phosphates is added dropwise to the column. Treatment carried out in this way results in complete ion exchange intercalation. This indicates that the reaction is also initiated with substoichiometric amounts of the nucleotides. The LDH may be used in the column as such or may be supported on an appropriate support material, for instance, the LDH may be embedded in resin beads. The addition of an aqueous mixture of nucleoside phosphates to a column of LDH (whether supported or not) leads to the elution of non-intercalated nucleoside phosphate(s) with the intercalated nucleoside phosphate(s) being retained in the LDH. The eluted nucleoside phosphate mixture may be recycled to the top of the column so that in a second and any subsequent passes through the column more of the desired nucleoside phosphate may be preferentially intercalated.

The nucleoside phosphate which is separated from the mixture by intercalation into the LDH can be readily recovered by the treatment of the LDH with an anion which intercalates between the layers in the LDH in preference to the nucleoside phosphate, thereby displacing the intercalated nucleoside phosphate from the LDH. A suitable anion for this purpose is carbonate although anions which intercalate more or less strongly than carbonate can be used. Carbonate is preferred, however, in most cases, on account of its strong capacity for binding with the LDH which allows the guest (i.e., intercalated) nucleoside phosphate to be recovered substantially quantitatively and since it potentially allows the material to be regenerated by calcining the carbonate intercalate and hydrating the resulting product. Typically, treatment of the LDH with carbonate to recover the nucleoside phosphate may involve treating the material with an aqueous solution of the soluble carbonate salt (e.g., sodium carbonate), preferably in a four to five molar excess compared to the intercalate compound, and stirring the mixture at about or above room temperature for up to several hours (e.g., at 20 to 80°C for from 1 to 20 hours).

The intercalated nucleoside phosphates may also be recovered from the LDH in other ways. For example, the LDH with the compound intercalated, may be treated in such a way as to break down the LDH and thereby free the compound (e.g., by treatment with dilute mineral acid, e.g., hydrochloric acid). This has the effect of protonating the phosphate and replacing the nucleotide with Cl⁻ ions in the LDH. Thus, the intercalated nucleoside phosphate may be removed by gradually protonating its negatively charged groups such that its retention within the LDH becomes energetically less favoured. Therefore, a further embodiment of the invention involves the recovery of the nucleoside phosphate from the LDH by a method which comprises treatment with an acid under conditions which cause protonation and deintercalation of the phosphate whilst leaving the layers of the material substantially intact.

The fact that the compound can be recovered from the material has clear advantages. Firstly, it allows the material to be regenerated, as mentioned above. Secondly, it means that a mixture of two nucleoside phosphates can be treated to provide substantial purification of each. For instance, a hypothetical mixture of A and B can be treated with the LDH to preferentially remove A perhaps with a minor amount of B from the mixture by intercalation into the material to leave behind B with perhaps a minor amount of A. The LDH with intercalated compound(s) may then be treated with an anion, such as carbonate, to release A with perhaps a minor amount of B which can be readily separated from the carbonate intercalate of the material, for example by filtration. Alternatively A with, perhaps a minor amount of B, could be released from its intercalate with the material by breaking down the material (e.g., by treatment with aqueous acid).

Reactivation of the LDH may be achieved by calcination, after the intercalated nucleoside phosphate has been displaced by treatment with a carbonate, such as Na₂CO₃ followed by hydration of the calcination product. In the case of [LiAl₂(OH)₆]⁺ materials reactivation can be achieved by treatment with a concentrated LiCl solution or HCl solution.

The method of the invention has been found to be particularly effective for the separation of cytidine-5'-monophosphate from aqueous equimolar mixtures of nucleoside phosphates, such as mixtures of cytidine-5'-monophosphate (CMP) and adenosine-5'-monophosphate (AMP) or mixtures of cytidine-5'-monophosphate (CMP) and guanosine-5'-monophosphate (GMP). In the former case, we have achieved a selectivity ratio of 80:20 (Na₂CMP:Na₂AMP) at 60°C using LiAl₂(OH)₆Cl.H₂O. In the latter case we have achieved a selectivity ratio of 75.6:24.4 (Na₂CMP:Na₂GMP) at 80°C using the same LiAl LDH.

The method of the invention involves, as an essential intermediate step, the production of a layered double hydroxide containing intercalated nucleoside phosphate. According to another aspect, therefore, the present invention provides a nucleoside phosphate intercalate of a layered double hydroxide. Such intercalates provide the means whereby one, or predominantly one, nucleoside phosphate can be separated from an aqueous solution containing a mixture of nucleoside phosphates. We have, using an LDH having layers comprised of [LiAl₂(OH)₆]⁺, formed intercalates of the nucleoside monophosphates adenosine monophosphate, guanosine monophosphate and cytidine monophosphate and of the triphosphate adenosine triphosphate.

It will be clear that the ability of a nucleoside phosphate to intercalate into a layered double hydroxide can be utilised in a method of separating a nucleoside phosphate from a solution containing it. In particular, a nucleoside phosphate may be separated from a solution containing it and one or more impurities (not being other nucleoside phosphates) which impurities do not intercalate into layered double hydroxides in preference to the nucleoside phosphate. By such a method, the purity of a nucleoside phosphate can be enhanced. In view of the excellent results obtained using layered double hydroxides containing monovalent metal cations the use of such layered double hydroxides forms an essential feature of such a method. Accordingly, there is further provided a method of separating a nucleoside phosphate from a solution containing it which method comprises the steps of (1) treating the solution with a layered double hydroxide having the general formula

[M^{I} ₍₁₋ₓ₎M^{III} ₓ(OH)₂]ⁿ⁺A^{z-} _{n/z}

where M^{I} is a monovalent metal cation, M^{III} is a trivalent metal cation, A is a displaceable anion, z is an integer, n = 2x - 1 and x is a positive number less than 1 which layered double hydroxide is optionally hydrated with a stoichiometric or a non-stoichiometric amount of water, whereby the nucleoside phosphate is preferentially intercalated into the layered double hydroxide
and (2) recovering the intercalated nucleoside phosphate from the layered double hydroxide.

According to such a method the layered double hydroxide will have a formula within the general formula given above. Preferably, however, the layered double hydroxide is one that has layers of [LiAl₂(OH)₆]⁻. The method is particularly useful for separating a nucleoside phosphate, such as adenosine -5'- monophosphate, citidine -5'- monophosphate or guanosine -5'- monophosphate, from a solution containing it and one or more impurities, not being other nucleoside phosphates, which impurities are substances which do not preferentially intercalate into the layered double hydroxide with respect to the nucleoside phosphate.

The solution containing the nucleoside phosphate may, according to this aspect of the invention be treated with particulate layered double hydroxide, preferably pre-treated with exposure to ultrasonic irradiation, in the manner described above. Recovery of the nucleoside phosphate from the intercalate compound formed by the intercalation of the nucleoside phosphate into the layered double hydroxide may be achieved using an anion, such as carbonate, which displaces the intercalated nucleoside phosphate or using a dilute acid, both of which recovery procedures are described in more detail above.

The ability to separate nucleoside phosphates by such simple procedures as disclosed herein is of value to industry and to laboratories alike where there is a need to purify such nucleotides.

The intercalate compounds containing intercalated nucleoside monophosphates exhibit remarkable thermal stability and, thus, may have utility as protective storage reservoirs or carriers of the nucleoside monophosphates.

### EXAMPLES

### NOTE

The formula mentioned in Examples 1 to 3 may deviate from the ideal formula. This can be explained as follows. The formulae mentioned only reflect the composition as determined by elemental microanalysis of the individual samples. They may, therefore, show variability from sample to sample since they are essentially nonstoichiometric compounds exhibiting large variations in their chemical composition. This variation in stoichiometry may be caused by depletion of Li in the host to compensate for the lower charge density of the guest species compared to the LDH.

### Example 1

Two equivalents of Na₂AMP in H₂O were reacted with [LiAl₂(OH)₆]Cl.H₂O at 80°C. Elemental microanalytical data, powder x-ray diffraction and thermal analysis of the resulting intercalate were consistent with full ion-exchange of the Cl⁻ ions in the layered double hydroxide. The powder XRD data for the intercalate can be indexed on a hexagonal unit (α=β=90°, γ=120°, a=b≈5.1Å). The observed interlayer separation was found to be 16.5Å-18.5Å and the chemical composition of the intercalate was determined as Li_{0.74}Al₂(OH)₆[AMP]_{0.37}.2.2H₂O.

### Example 2

The procedure described in Example **1** was repeated but using Na₂CMP instead of Na₂AMP. The observed interlayer separation in the resulting intercalate compound was found to be 14.5Å and the chemical composition of the intercalate was determined as Li_{0.23}Al₂ (OH)₆[CMP]_{0.12}.0.9H₂O.

### Example 3

The procedure described in Example 1 was repeated but using Na₂GMP instead of Na₂AMP. The observed interlayer separation in the resulting intercalate compound was found to be 18.5Å and the chemical composition of the intercalate was determined as Li_{0.31}Al₂(OH)₆[GMP]_{0.15}.1.7H₂O.

### Example 4

The procedure described in Example 1 was repeated but using the sodium salt of ATP. The observed interlayer separation in the resulting intercalate compound was found to be 22.6 Å. The chemical composition of the intercalate formed was not determined.

### X-Ray Diffraction Studies (on the products formed in Examples 1 to 4)

Absence of (001)-host reflections in most of the product samples confirmed complete intercalation with the nucleotide, which is rather uncommon for ion exchange with large organic molecules, where a small percentage of the initial guest anion is usually retained. The absence of Cl⁻ anions was confirmed by elemental analysis. However, incomplete ion exchange was also randomly observed in several CMP- and, even more pronounced, GMP-intercalates, and the occurrence of a remaining Cl⁻ phase could be minimised, yet not completely prevented, by ultrasonication of the pristine LDH.

In general, products of average crystallinity were obtained for the AMP guest, rather poor crystallinity was observed for the GMP and ATP guests, whereas very broad reflections of low intensity, were symptomatic of the CMP intercalate, which could only be slightly improved by extension of the reaction time to 2 days. The relatively low crystallinity can be accounted for by a small particle size, since the breadth of the peaks is inversely proportional to the particle size. Additionally, disorder in the stacking sequence along the c axis or orientational flexibility of the interlayer anions might contribute as well, visible in the absence of basal (001)-reflections higher than the second harmonic, (004). An angular offset between successive sheets (turbostratic structure) can be seen from the weakening or total absence of the (hk0)-and (Okl)-reflections. This vanishing of layer registry might be attributed to relatively weak bonding between the guest anions and layers.

Another prominent feature of the CMP, GMP, and, to a lesser extent, AMP powder patterns is the presence of the reflections attributable to Al(OH)₃ at 2θ values around 18.5 and 20.5. In some nucleoside monophosphate patterns, small amounts of Al₂O₃ are also present. This feature was also observed when recovering the initial LDH after stirring it with a mixture of nucleotides at a temperature insufficiently high for intercalation (40°C). Elemental analysis of a CMP-reaction solution indicated the deintercalation of about two thirds of the Li cations, originally embedded in the Al(OH)₃ layers, which was in accord with the observed Li : Al ratio of about 1 : 6 in the intercalation product. This can be accounted for by the reduction of a mismatch in charge density of the layers and the nucleotide. A layer charge density of 0.044 /Å² (unit charge / 22.5 Å²) can be calculated on the basis of a hexagonal unit cell with a = b ≈ 5.1 Å, with one Li cation per hexagon. The charge density of the nucleotides can only be determined approximately by calculating the area occupied by a molecule in a crystal (derived from the parameters of the unit cell), taking into account the Van-der-Waals surface and the approximate height of the nucleoside monophosphate in the most probable arrangement within the layers. The charge densities were found to be in the range 0.033 / Å² - 0.040 / Å², hence being smaller than the layer charge density. Since the removal of the Li cations results in less positive layer charge and, thus, in a reduction in layer charge density, this might enhance the Li-deintercatation.

The XRD data could also be interpreted in terms of a separate Al(OH)₃ phase located on the crystal surface. This may result from salt formation between Li⁺ and NP-anions, which is likely to be initiated predominantly at the surface regions. This also accounts for the observation of an Al(OH)₃ phase in the absence of intercalation. However, it is known that LDH intercalates of low crystallinity often exhibit a low M^{II}/M^{III} ratio, coincident with a depletion of the divalent metal during the intercalation process. Although the charge density in these cases increases, it has been suggested that the trivalent metal enrichment could be a general kinetic effect, and this consideration might also apply to the aluminium enrichment in this case.

The basic spacings for the AMP-intercalates were found to vary between 16.5 Å and 18.5 Å, whereas the (002)-reflections for the other NPs deviate significantly less from the mean value given in Table 1.

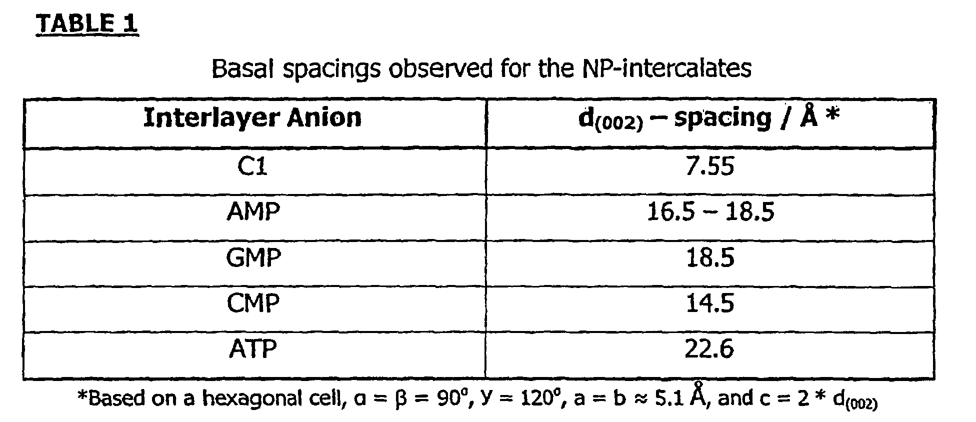

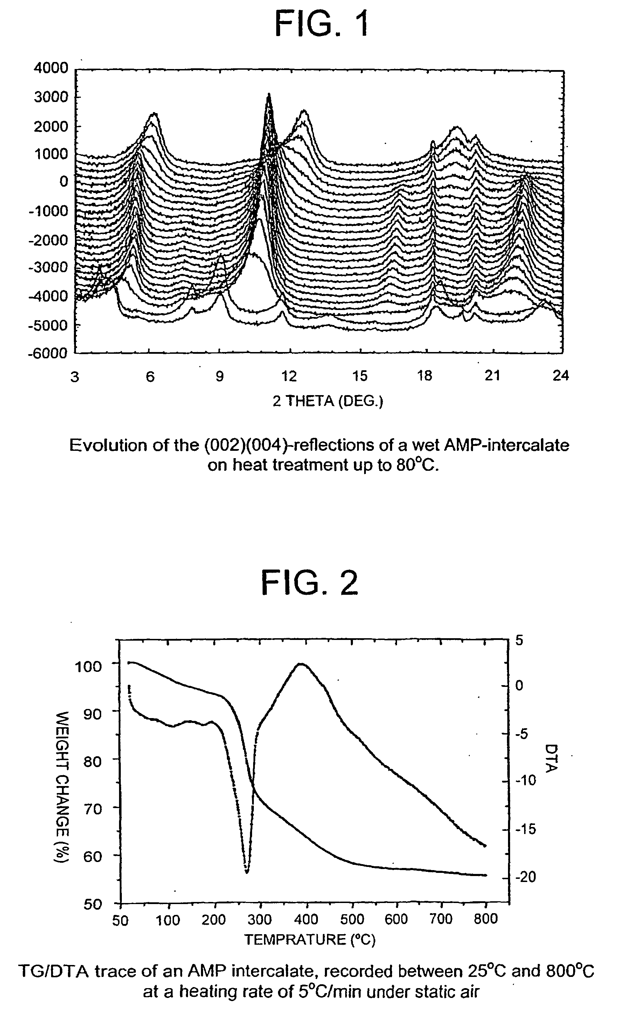

This shift in the basal spacing was presumed to depend on the hydration state of the sample, and hence on the preparation and drying conditions applied, rather than on compositional changes or reorientation of the guest at the same state of hydration. To confirm this interpretation, XRD powder patterns were recorded on a wet AMP-intercalate, which was allowed to dry while heating from 28 C to 80°C (Figure 1). From this, it was seen that the gradual shift in the (001)-reflections toward higher 2θ-values on heating are in line with a decrease of the gallery height due to the loss of interlayer water. At 28°C, the (002)-reflection is split, consistent with basal spacings of 22.8 Å and 20.6 Å, which might be attributed to two differently hydrated phases. At 80°C, only one phase is detectable, exhibiting a d-spacing of 14.3 Å, which corresponds to almost complete dehydration of the sample. However, simultaneous reorientation of the molecules While heating, affecting the basal spacing, cannot be excluded.

### Thermal Characteristics of the Intercalates

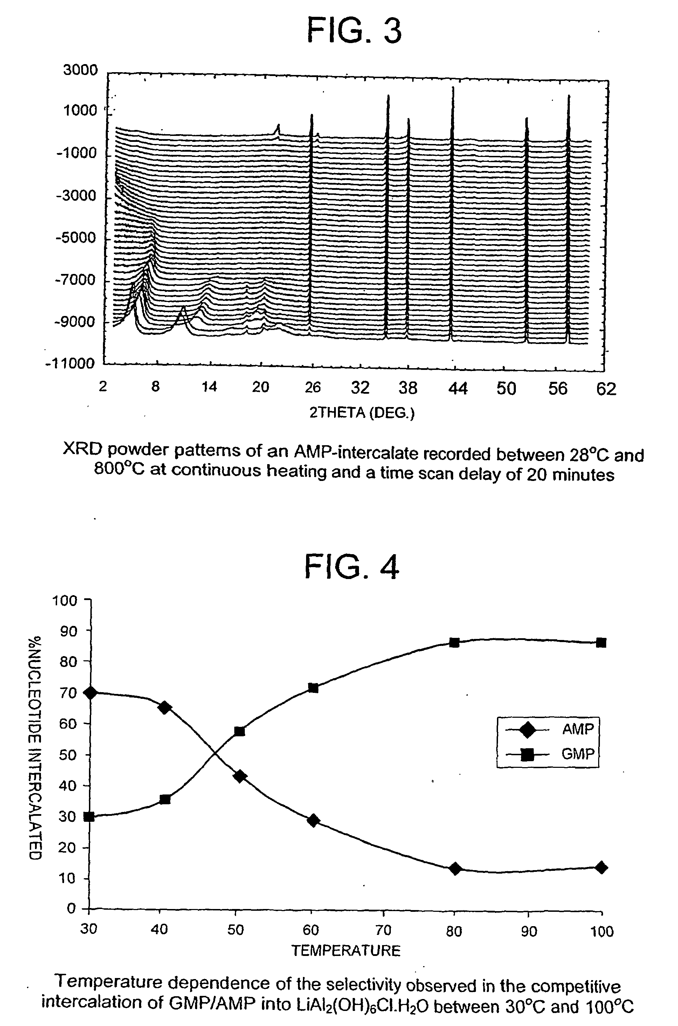

The thermal characteristics of three nucleoside monophosphate samples were studied by thermogravimetric analysis. Gradual chemical changes during heating were visualised by an *in situ* diffraction study on an AMP-intercalate under moisture-saturated air at increasing temperatures up to 800°C. The TG/DTA trace of an AMP sample, dried *in vacuo* prior to data acquisition, and the temperature evolution of the diffraction patterns are shown in Figures 2 and 3, respectively.

Three mass loss regions are observed in the TG trace. The first loss (≈ 6%) occurs in a broad range, owing to the loss of physisorbed external surface water (typically between 40°C and 80°C), and the loss of ≈1.5 moles of interlamellar water (between 80°C and 200°C), yielding the dehydrated sample LiAl_{2.7}(OH)_{8.1}[AMP]_{0.5}. The loss of intercalated water is accompanied by a decrease in the interlayer repeat distance from 17.5 Å to 14 Å, and the gradual decay of the (004)-Bragg reflection indicates its association with the presence and arrangement of intragallery water.

The onset of the second significant mass loss (≈19%), which is recorded at 220°C and continues up to 290°C, can be ascribed to the dehydroxylation of the layers, yielding a sample of the idealised composition LiAl_{2.7}O_{4.1}[AMP]_{0.5}. However, XRD data suggest that the loss of interlayer water continues up to the complete disappearance of the (004)-reflection at - 260°C, and the incomplete loss of the (002)-reflection indicates that a minimum of structural order is maintained up to 450°C. This is rather atypical and might be due to the stabilisation of a small fraction of the layered structure by the organic anion, e.g. by grafting of the guest into the layers.

Decomposition of the organic material, which is indicated by the combustion endotherm centred at 410°C, occurs gradually between 300°C and 500°C and is accompanied by a weight loss of about 18% and complete collapse of the layered structure. Calcination of the AMP-intercalate results in a mixed metal oxide/phosphate phase with the approximate composition 0.5Li₂O · 0.5AlPO₄ · 1.1Al₂O₃. Crystalline corundum, which is already present in small amounts in the uncalcined intercalate (*vide supra)* is the prominent phase at high temperatures.

### Example 5

Competition reactions were performed in which [LiAl₂(OH)₆]Cl.H₂O was reacted with a two-component mixture of nucleoside phosphates at various temperatures and the relative intercalation preferences were determined. The products were characterised by X-ray diffraction studies.

An aqueous solution (10ml) containing a mixture of two different nucleoside phosphates, each at a concentration of 0.07M, was added to 10ml of an aqueous suspension of [LiAl₂(OH)₆]Cl.H₂O (0.035M) and the mixture was stirred at a given temperature within the range of 20° to 100°C for 2 hours. The aqueous suspension, prior to the addition of the nucleoside phosphate solution, had been ultrasonicated for 30 minutes. The percentages of each nucleoside phosphate intercalated in the LDH during the competition reactions were determined as follows.

Following the intercalation reaction the LDH containing the intercalated nucleoside phosphates was separated from the reaction solution by filtration and washed with deionised water. The LDH was then treated with 10ml of D₂O solution containing Na₂CO₃ in a four fold excess compared to the LDH intercalate and stirred for 20h at 80°C to cause deintercalation of the nucleoside phosphates by the carbonate anion. The resulting mixture was filtered and the aqueous phase containing the deintercalated nucleoside phosphates was analysed using ¹H NMR. On repeating the experiments, the selectivity values were found to be reproducible within ^{±} 3%. The results of the experiments are shown in the following Table 2.

**TABLE 2**

| Competitive intercalation reactions of nucleoside phosphates (NP) into LiAl₂(OH)₆Cl·H₂0 at temperatures between 20°C (room temperature) and 100°C. | | | |
|---|---|---|---|
| **NP Combination** | **T (°C)** | **NP intercalated preferentially** | **Selectivity** |
| Na₂AMP + Na₂CMP | RT | No intercalation | - |
| | 40 | No intercalation | - |
| | 60 | CMP | 80.0 : 20.0 |
| | 80 | CMP | 80.5 : 19.5 |
| | 100 | CMP | 79.7 : 20.3 |
| Na₂CMP + Na₂GMP | RT | No intercalation | - |
| | 40 | No intercalation | - |
| | 60 | CMP | 72.4 : 27.6 |
| | 80 | CMP | 75.6 : 24.4 |
| | 100 | CMP | 73.7 : 26.3 |
| Na₂AMP + Na₂GMP | RT | No intercalation | - |
| | 30 | AMP | 69.4 : 30.6 |
| | 40 | AMP | 64.5 : 35.5 |
| | 50 | GMP | 57.1 : 42.9 |
| | 60 | GMP | 71.4 : 28.6 |
| | 80 | GMP | 86.5 : 13.5 |
| | 100 | GMP | 86.0 : 14.0 |
| Na₂AMP + Na₂ATP | RT | No intercalation | |
| | 40 | ATP | |
| | 60 | ATP | |
| | 80 | ATP | |
| | 100 | Amorphous compound recovered | |

As shown in Table 2, LiAl₂(OH)₆Cl.H₂O was able to preferentially intercalate Na₂CMP from an equimolar aqueous solution of Na₂CMP and Na₂AMP at a selectivity ratio of 80.5:19.5 at a reaction temperature of 80°C. At 80°C, the LDH was able to preferentially intercalate Na₂CMP from an equimolar aqueous solution of Na₂CMP and Na₂GMP at a selectivity ratio of 75.6 to 24.4.

Remarkably, the LDH exhibits a strong preference for intercalating Na₂GMP from an equimolar aqueous solution of Na₂GMP and Na₂AMP at higher temperatures. For example, at a reaction temperature of 100°C the selectivity ratio Na₂GMP:Na₂AMP was 86:14. However, at a reaction temperature of 30°C the intercalation preference was for Na₂AMP; the selectivity ratio being 30.6:69.4

Formulae for the products of the competition reactions at 80°C were calculated according to the selectivities obtained, and confirmed by elemental analysis. These are shown in Table 3 below.

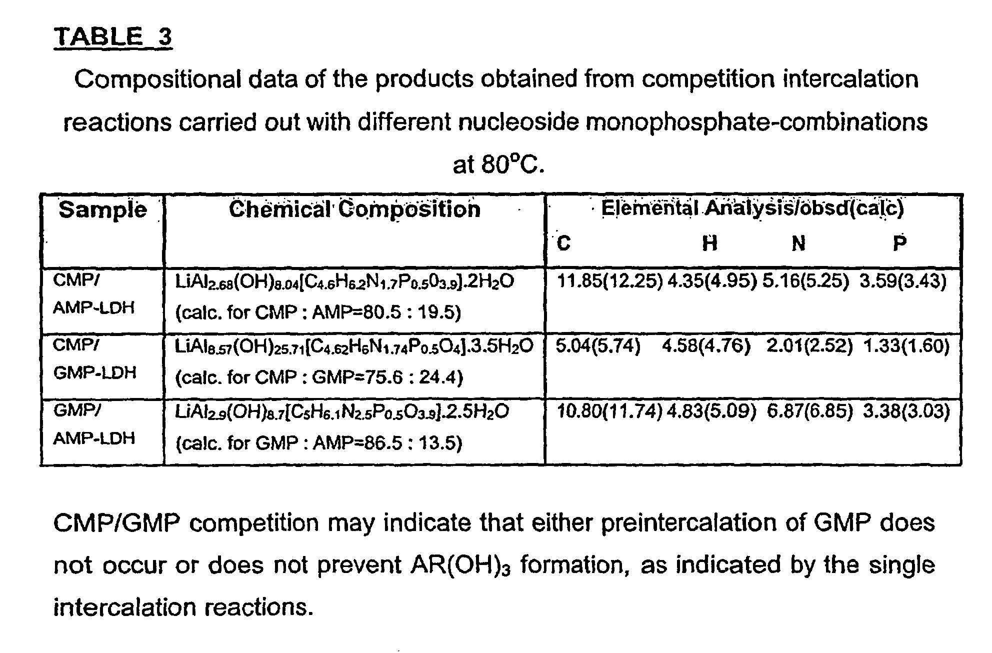

### X-Ray Diffraction Studies

As shown above, CMP is intercalated preferentially over both purine-nucleoside monophosphates by LiAl₂(OH)₆Cl.H₂O at all temperatures, which contrasts with the difficulties observed for the single intercalation of CMP compared to AMP and, to a lesser extent, GMP. The basal spacing revealed by the powder patterns is identical with that expected for the pure CMP phase. Since a significantly larger d-spacing must be assumed for AMP compared to CMP, the accommodation of the cointercalated AMP molecules within the same interlayer sites as CMP is questionable. It appears to be more likely that either a separate AMP phase is formed, which is too small to be detected, or that the interlayer domains filled with AMP are not detectable because they are randomly stacked and encapsulated between the more abundant interlayer spaces occupied by CMP, hence not giving rise to a pronounced (001)-reflection series.

The result of the AMP/GMP competition reaction is largely dependent on the reaction temperature, an inversion of the intercalation preference being observed at intermediate temperatures as can be seen from Figure 4.

At 30°C and 40°C, only the AMP phase, and at 80°C and 100°C only the GMP phase, is detectable in the powder patterns, whereas the (002)/(004)-peaks are split at 50°C and 60°C and can be attributed to separate nucleoside monophosphate phases. The presence of both phases at intermediate temperatures and low ion selectivities suggest that the nudeoside monophosphates occupy different interlayer domains and that interactions between the nucleotides, such as hydrogen bonding, are negligible.

At all temperatures, the powder patterns collected for the AMP/ATP competition reactions are in agreement with an ATP-intercalated phase. However, at 40°C and 60°C, minor reflections were found which could not be attributed to a Cl⁻ or AMP phase, and at 100°C an amorphous product was recovered. This might reflect the lower thermal stability of ATP and its sensitivity towards hydrolysis, which could cause the collapse of the intercalate layers or give rise to cointercalation of hydrolysis products such as pyrophosphate, P₂O₇⁴⁻. However, selectivity data could not be acquired, since hydrolysis of the ATP molecules, even under very mild conditions (low temperature, short reaction time) could not be prevented during the deintercalation process, and NMR data confirmed that AMP and another species, presumably ADP, were the main products obtained.

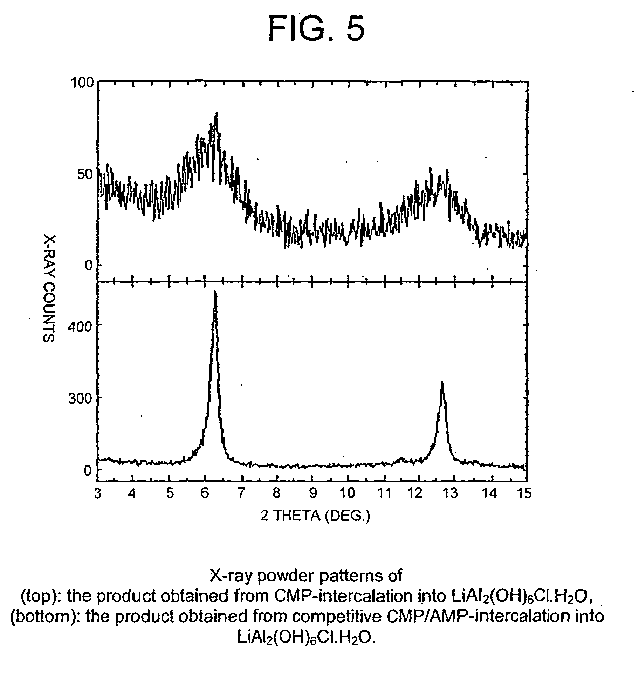

Another striking aspect of the powder patterns is the difference in crystallinity at different temperatures and for different nucleoside phosphate combinations. The products obtained in CMP/AMP competition reactions at 80°C exhibit remarkable crystallinity and phase purity (almost no Al(OH)₃ detected), which is several degrees higher than that for CMP/GMP samples and CMP-intercalates at the same temperature, as can be seen in Figure 5.

However, at temperatures other than 80°C, the crystallinity is poor for both CMP/AMP and CMP/GMP combinations, and concomitant Al(OH)₃ formation becomes the main feature. In contrast, relatively sharp reflections observed for the GMP/AMP and ATP/AMP samples indicate good crystallinity at all temperatures.

The powder patterns indicate full exchange of the Cl⁻ anions by the nucleoside phosphates, which was confirmed by elemental analysis.

### Example 6

In order to elucidate if the intercalation preference and degree of selectivity observed for LiAl-Cl are retained when using different LDHs, various LDH precursors in the Cl⁻- or NO⁻₃ form were prepared and reacted with an equimolar aqueous solution of Na₂ CMP and Na₂ AMP according to the procedure described in Example 4, at a reaction temperature of 80°C. The results are shown in the following Table 4.

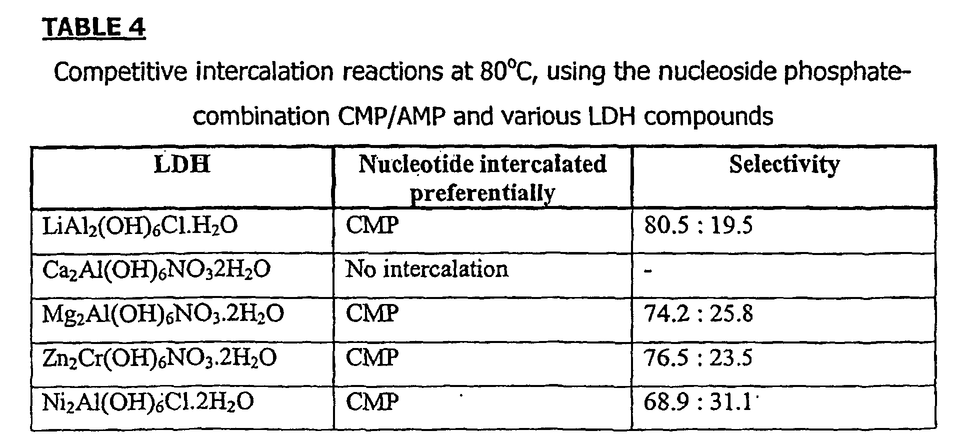

According to the XRD data and quantitative ¹H NMR analysis, those hydrotalcite-like materials for which intercalation was observed also exhibit intercalation preference for CMP. Intercalation did not occur for the CaAl-LDH, yet a new phase was observed, which might be attributed to salt formation between the nucleotides and layer Ca²⁺ ions with concomitant host destruction.

The selectivities observed for the different hosts roughly lie within the same order of magnitude, thus reflecting the same reaction behaviour of the structurally related LDH materials. Minor differences may result from different layer charge densities, and proportionality between charge density and CMP-selectivity might tentatively be stated, ranking LiAl-LDH (with the highest charge density) as the most selective precursor.

## Claims

1. A method of separating a first nucleoside phosphate from a solution containing it and at least a second nucleoside phosphate which method comprises treating the solution with a layered double hydroxide whereby the first nucleoside phosphate is preferentially intercalated into the layered double hydroxide and then recovering the intercalated nucleoside phosphate from the layered double hydroxide.

2. A method according to claim 1, wherein the solution is an aqueous solution.

3. A method according to claim 1 or claim 2, wherein the temperature of the solution treated with a layered double hydroxide is in the range of from 30 to 100°C.

4. A method according to any one of claims 1 to 3, wherein the layered double hydroxide is a compound having the general formula
[M^{I} ₍₁₋ₓ₎M^{III} ₓ(OH)₂]ⁿ⁺A^{z-} _{n/z}
wherein M^{I} is a monovalent metal cation;
M^{III} is a trivalent metal cation; and
A is a displaceable anion, z is an integer, n = 2x-1, and x is a number less than 1, which compound is optionally hydrated with a stoichiometric or non-stoichiometric amount of water.

5. A method according to claim 4, wherein M^{I} is Li and M^{III} is Al.

6. A method according to claim 4 or claim 5, wherein A is selected from OH, F, Cl, Br, I, SO₄ or NO₃.

7. A method according to claim 6, wherein the layered double hydroxide is LiAl₂(OH)₆Cl.H₂O.

8. A method according to any one of claims 1 to 3, wherein the layered double hydroxide is a compound having the general formula
[M^{II} ₍₁₋ₓ₎M^{III} ₓ(OH)₂]^{x+}A^{z-} _{x/z}
where M" is a divalent metal cation;
M^{III} is a trivalent metal cation;
A is a displaceable anion; z is an integer and x is a number less than 1, which compound is optionally hydrated with a stoichiometric or non-stoichiometric amount of water.

9. A method according to claim 8, wherein M" is Mg or Ni, and M^{III} is Al.

10. A method according to claim 8, wherein M" is Zn and M^{III} is Cr.

11. A method according to any one of claim 8 to 10, wherein A is selected from OH, F, Cl, Br, I, SO₄ or NO₃.

12. A method according to claim 11, wherein the layered double hydroxide is selected from Mg₂Al(OH)₆NO₃.2H₂O, Zn₂Cr(OH)₆NO₃.2H₂O and Ni₂Al(OH)₆Cl.2H₂O.

13. A method according to any one of claims 1 to 12, whereby a nucleoside monophosphate is separated from a solution containing it and at least a second nucleoside monophosphate.

14. A method according to claim 13, for separating cytidine-5'-monophosphate from adenosine-5'-monophosphate.

15. A method according to claim 13, for separating cytidine-5'-monophosphate from guanosine-5'-monophosphate.

16. A method according to claim 13, for separating guanosine-5'-monophosphate from adenosine-5'-monophosphate.

17. A method according to any one of claims 1 to 16, wherein particulate layered double hydroxide is mixed with the aqueous mixture containing the first and the second nucleoside phosphates and is separated therefrom after the intercalation reaction.

18. A method according to any one of claims 1 to 16, wherein the aqueous mixture containing the first and the second nucleoside monophosphates is contacted, either in a batch or flow process, with particulate layered double hydroxide.

19. A method according to any one of claims 1 to 18, wherein the intercalated nucleoside phosphate is recovered from the layered double hydroxide by treatment of the layered double hydroxide with a solution of an anion which intercalates into the layered double hydroxide in preference to the nucleoside phosphate.

20. A method according to claim 19, wherein the anion is carbonate.

21. A method according to any one of claims 1 to 18, wherein the intercalated nucleoside phosphate is recovered from the layered double hydroxide by treatment of the layered double hydroxide with an acid under conditions which cause protonation and deintercalation of the nucleoside phosphate whilst leaving the layers of the layered double hydroxide substantially intact.

22. A nucleoside phosphate intercalate of a layered double hydroxide having, before intercalation, layers of [M^{I}₍₁₋ₓ₎M^{III}ₓ(OH)₂]ⁿ⁺, wherein M' is a monovalent metal cation, M^{III} is a trivalent metal cation, x is a number less than 1 and n = 2x-1, between which layers the nucleoside phosphate is intercalated.

23. An intercalate according to claim 22, wherein the layered double hydroxide used to form the intercalate has layers of [LiAl₂(OH)₆]⁺.

24. An intercalate according to claims 22 or claims 23, wherein the nucleoside phosphate is a monophosphate selected from cytidine-5'-monophosphate, guanosine-5'-monophosphate, and adenosine-5'-monophosphate.

25. An intercalate according to claim 22 or claim 23, wherein the nucleoside phosphate is adenosine triphosphate.

26. A method of separating a nucleoside phosphate from a solution containing it which method comprises the steps of (1) treating the solution with a layered double hydroxide having the general formula
[M^{I} ₍₁₋ₓ₎M^{III} ₓ(OH)₂]ⁿ⁺A^{z-} _{*n*/*z*}
wherein M^{I} is a monovalent metal cation; M^{III} is a trivalent metal cation; and A is a displaceable anion, z is an integer, n = 2x - 1, and x is a number less than 1, which layered double hydroxide is optionally hydrated with a stoichiometric or non-stoichiometric amount of water, whereby the nucleoside phosphate is preferentially intercalated into the layered double hydroxide and (2) recovering the intercalated nucleoside phosphate from the layered double hydroxide.

27. A method according to claim 26, wherein the layered double hydroxide has layers of [LiAl₂(OH)₆]⁺.

28. A method according to either claim 26 or claim 27, wherein the nucleoside phosphate is a monophosphate selected from adenosine -5'- monophosphate, cytidine -5'- monophosphate and guanosine -5'- monophosphate.

29. A method according to any one of claims 26 to 28, wherein particulate layered double hydroxide is mixed with the solution containing the nucleoside phosphate and is separated therefrom after the intercalation reaction.

30. A method according to any one of claims 26 to 28, wherein the solution containing the nucleoside phosphate is passed through a body of particulate layered double hydroxide.

31. A method according to any one of claims 26 to 30, wherein prior to the treatment of the solution containing the nucleoside phosphate with the layered double hydroxide, the layered double hydroxide is treated with ultrasonic irradiation.

32. A method according to any one of claims 26 to 31, wherein the intercalated nucleoside phosphate is recovered from the layered double hydroxide in which it is intercalated by treatment of the layered double hydroxide with a solution of an anion which intercalates into the layered double hydroxide in preference to the nucleoside phosphate so as to displace the nucleoside phosphate from the layered double hydroxide.

33. A method according to claim 32, wherein the anion is carbonate.

34. A method according to any one of claims 26 to 31, wherein the intercalated nucleoside phosphate is recovered from the layered double hydroxide in which it is intercalated by treatment of the layered double hydroxide with an acid under conditions which cause protonation and deintercalation of the nucleoside phosphate whilst leaving the layers of the layered double hydroxide substantially intact.

## Patentansprüche

1. Verfahren zur Trennung eines ersten Nukleosidphosphats aus einer Lösung, die dieses und zumindest ein zweites Nukleosidphosphat enthält, wobei das Verfahren die Behandlung der Lösung mit einem Schichtdoppelhydroxid, wobei das erste Nukleosidphosphat vorzugsweise in das Schichtdoppelhydroxid eingelagert wird, und dann die Wiedergewinnung des eingelagerten Nukleosidphosphats aus dem Schichtdoppelhydroxid umfaßt.

2. Verfahren nach Anspruch 1, wobei die Lösung eine wässerige Lösung ist.

3. Verfahren nach Anspruch 1 oder Anspruch 2, wobei die Temperatur der Lösung, die mit einem Schichtdoppelhydroxid behandelt wurde, im Bereich von 30 bis 100 °C liegt.

4. Verfahren nach einem der Ansprüche 1 bis 3, wobei das Schichtdoppelhydroxid eine Verbindung mit der allgemeinen Formel
[M^{I} ₍₁₋ₓ₎M^{III} ₓ(OH)₂]ⁿ⁺A^{z-} _{n/z}
ist, worin M^{I} ein einwertiges Metallkation ist;
M^{III} ein dreiwertiges Metallkation ist; und
A ein austauschbares Anion ist, z eine ganze Zahl ist, n = 2x - 1, und x eine Zahl von weniger als 1 ist, wobei die Verbindung gegebenenfalls mit einer stöchiometrischen oder nicht stöchiometrischen Menge an Wasser hydratisiert ist.

5. Verfahren nach Anspruch 4, wobei M^{I} Li ist und M^{III} Al ist.

6. Verfahren nach Anspruch 4 oder Anspruch 5, wobei A aus OH, F, Cl, Br, I, SO₄ oder NO₃ ausgewählt ist.

7. Verfahren nach Anspruch 6, wobei das Schichtdoppelhydroxid LiAl₂(OH)₆Cl · H₂O ist.

8. Verfahren nach einem der Ansprüche 1 bis 3, wobei das Schichtdoppelhydroxid eine Verbindung mit der allgemeinen Formel
[M^{II} ₍₁₋ₓ₎M^{III} ₓ(OH)₂]^{x+}A^{z-} _{o/z}
ist, worin M^{II} ein zweiwertiges Metallkation ist;
M^{III} ein dreiwertiges Metallkation ist;
A ein austauschbares Anion ist, z eine ganze Zahl ist und x eine Zahl von weniger als 1 ist, wobei die Verbindung gegebenenfalls mit einer stöchiometrischen oder nicht stöchiometrischen Menge an Wasser hydratisiert ist.

9. Verfahren nach Anspruch 8, wobei M^{II} Mg oder Ni ist und M^{III} Al ist.

10. Verfahren nach Anspruch 8, wobei M^{II} Zn ist und M^{III} Cr ist.

11. Verfahren nach einem der Ansprüche 8 bis 10, wobei A aus OH, F, Cl, Br, I, SO₄ oder NO₃ ausgewählt ist.

12. Verfahren nach Anspruch 11, wobei das Schichtdoppelhydroxid aus Mg₂Al(OH)₆NO₃ · 2H₂O, Zn₂Cr(OH)₆NO₃ · 2H₂O und Ni₂Al(OH)₆Cl · 2H₂O ausgewählt ist.

13. Verfahren nach einem der Ansprüche 1 bis 12, wobei ein Nukleosidmonophosphat aus einer Lösung getrennt wird, die dieses und zumindest ein zweites Nukleosidmonophosphat enthält.

14. Verfahren nach Anspruch 13 zur Trennung von Cytidin-5'-monophosphat aus Adenosin-5'-monophosphat.

15. Verfahren nach Anspruch 13 zur Trennung von Cytidin-5'- monophosphat aus Guanosin-5'-monophosphat.

16. Verfahren nach Anspruch 13 zur Trennung von Guanosin-5'-monophosphat aus Adenosin-5'-monophosphat.

17. Verfahren nach einem der Ansprüche 1 bis 16, wobei partikuläres Schichtdoppelhydroxid mit dem wässerigen Gemisch, das das erste und das zweite Nukleosidphosphat enthält, gemischt und nach der Einlagerungsreaktion daraus abgetrennt wird.

18. Verfahren nach einem der Ansprüche 1 bis 16, wobei das wässerige Gemisch, das das erste und das zweite Nukleosidphosphat enthält, entweder in einem diskontinuierlichen oder einem Fließverfahren mit partikulärem Schichtdoppelhydroxid kontaktiert wird.

19. Verfahren nach einem der Ansprüche 1 bis 18, wobei das eingelagerte Nukleosidphosphat aus dem Schichtdoppelhydroxid durch die Behandlung des Schichtdoppelhydroxids mit einer Lösung eines Anions, das sich lieber in das Schichtdoppelhydroxid als in das Nukleosidphosphat einlagert, wiedergewonnen wird.

20. Verfahren nach Anspruch 19, wobei das Anion Carbonat ist.

21. Verfahren nach einem der Ansprüche 1 bis 18, wobei das eingelagerte Nukleosidphosphat aus dem Schichtdoppelhydroxid durch die Behandlung des Schichtdoppelhydroxids mit einer Säure unter Bedingungen, die Protonierung und Deinterkalation des Nukleosidphosphats verursachen, während die Schichten des Schichtdoppelbydroxid im wesentlichen intakt bleiben, wiedergewonnen wird.

22. Nukleosidphosphatinterkalat eines Schichtdoppelhydroxids, das vor der Interkalation Schichten aus [M^{I}₍₁₋ₓ₎M^{III}ₓ(OH)₂]ⁿ⁺ aufweist, wobei M^{I} ein einwertiges Metallkation ist, M^{III} ein dreiwertiges Metallkation ist, x eine Zahl von weniger als 1 ist und n = 2x - 1, wobei das Nukleosidphosphat zwischen den Schichten eingelagert wird.

23. Interkalat nach Anspruch 22, wobei das zur Bildung des Interkalats verwendete Schichtdoppelhydroxid Schichten aus [LiAl₂(OH)₆]⁺ aufweist.

24. Interkalat nach Anspruch 22 oder Anspruch 23, wobei das Nukleosidphosphat ein Monophosphat ist, ausgewählt aus Cytidin-5'-monophosphat, Guanosin-5'-monophosphat und Adenosin-5'-monophosphat.

25. Interkalat nach Anspruch 22 oder Anspruch 23, wobei das Nukleosidphosphat Adenosintriphosphat ist.

26. Verfahren zur Trennung eines Nukleosidphosphats aus einer Lösung, die dieses enthält, wobei das Verfahren die Schritte
(1) Behandeln der Lösung mit einem Schichtdoppelhydroxid mit der allgemeinen Formel
[M^{I} ₍₁₋ₓ₎M^{III} ₓ(OH)₂]ⁿ⁺A^{z-} _{n/z}
worin M^{I} ein einwertiges Metallkation ist;
M^{III} ein dreiwertiges Metallkation ist; und
A ein austauschbares Anion ist,
z eine ganze Zahl ist, n = 2x - 1, und x eine Zahl von weniger als 1 ist,
wobei das Schichtdoppelhydroxid gegebenenfalls mit einer stöchiometrischen oder nicht stöchiometrischen Menge an Wasser hydratisiert ist,
wobei das Nukleosidphosphat vorzugsweise in das Schichtdoppelhydroxid eingelagert wird und
(2) Wiedergewinnung des eingelagerten Nukleosidphosphats aus dem Schichtdoppelhydroxid umfaßt.

27. Verfahren nach Anspruch 26, wobei das Schichtdoppelhydroxid Schichten aus [LiAl₂(OH)₆]⁺ aufweist.

28. Verfahren nach Anspruch 26 oder Anspruch 27, wobei das Nukleosidphosphat ein Monophosphat ist, ausgewählt aus Adenosin-5'-monophosphat, Cytidin-5'-monophosphat und Guanosin-5'-monophosphat.

29. Verfahren nach einem der Ansprüche 26 bis 28, wobei das partikuläre Schichtdoppelhydroxid mit der Lösung, die das Nukleosidphosphat enthält, gemischt und nach der Einlagerungsreaktion daraus abgetrennt wird.

30. Verfahren nach einem der Ansprüche 26 bis 28, wobei die Lösung, die das Nukleosidphosphat enthält, durch einen Körper aus partikulärem Schichtdoppelhydroxid geführt wird.

31. Verfahren nach einem der Ansprüche 26 bis 30, wobei vor der Behandlung der Lösung, die das Nukleosidphosphat enthält, mit dem Schichtdoppelhydroxid das Schichtdoppelhydroxid mit Ultraschallbestrahlung behandelt wird.

32. Verfahren nach einem der Ansprüche 26 bis 31, wobei das eingelagerte Nukleosidphosphat aus dem Schichtdoppelhydroxid, in das es durch die Behandlung des Schichtdoppelhydroxids mit einer Lösung eines Anions, das sich lieber in das Schichtdoppelhydroxid als in das Nukleosidphosphat einlagert, wiedergewonnen wird, so das das Nukleosidphosphat aus dem Schichtdoppelhydroxid verdrängt wird.

33. Verfahren nach Anspruch 32, wobei das Anion Carbonat ist.

34. Verfahren nach einem der Ansprüche 26 bis 31, wobei das eingelagerte Nukleosidphosphat aus dem Schichtdoppelhydroxid, in das es eingelagert ist, durch die Behandlung des Schichtdoppelhydroxids mit einer Saure unter Bedingungen, die Protonierung und Deinterkalation des Nukleosidphosphats verursachen, während die Schichten des Schichtdoppelhydroxids im wesentlichen intakt bleiben, wiedergewonnen wird.

## Revendications

1. Procédé de séparation d'un premier nucléoside-phosphate d'une solution le contenant et d'au moins un deuxième nucléoside-phosphate, lequel procédé comprend le traitement de la solution avec un double hydroxyde en couches, grâce à quoi le premier nucléoside-phosphate est de préférence intercalé dans le double hydroxyde en couches puis la récupération du nucléoside-phosphate intercalé du double hydroxyde en couches.

2. Procédé selon la revendication 1, dans lequel la solution est une solution aqueuse.

3. Procédé selon la revendication 1 ou 2, dans lequel la température de la solution traitée avec un double hydroxyde en couches est comprise dans la plage de 30 à 100°C.

4. Procédé selon l'une quelconque des revendications 1 à 3, dans lequel le double hydroxyde en couches est un composé ayant la formule générale suivante:
[M^{I} ₍₁₋ₓ₎M^{III}x(OH)₂]ⁿ⁺A^{z-} _{n/z}
où M^{I} est un cation métallique monovalent ;
M^{III} est un cation métallique trivalent ; et
A est un anion déplaçable, z est un nombre entier, n = 2x-1, et x est un nombre inférieur à 1, lequel le composé est hydraté de façon facultative à l'aide d'une quantité d'eau stoechiométrique ou non stoechiométrique.

5. Procédé selon la revendication 4, dans lequel M^{I} est du Li et M^{III} est Al.

6. Procédé selon la revendication 4 ou 5, dans lequel A est choisi parmi OH, F, Cl, Br, I, SO₄ ou NO₃.

7. Procédé selon la revendication 6, dans lequel le double hydroxyde en couches est LiAl₂(OH)₆Cl.H₂O,

8. Procédé selon l'une quelconque des revendications 1 à 3, dans lequel le double hydroxyde en couches est un composé ayant la formule générale suivante :
[M^{II} ₍₁₋ₓ₎M^{III}x(OH)₂]^{x+}A^{z-} _{x/z}
où M^{II} est un cation métallique divalent ;
M^{III} est un cation métallique trivalent ;
A est un anion déplaçable ; z est un nombre entier et x est un nombre inférieur à 1, lequel le composé est hydraté de façon facultative à l'aide d'une quantité d'eau stoechiométrique ou non stoechiométrique.

9. Procédé selon la revendication 8, dans lequel M^{II} est Mg ou Ni, et M^{III} est Al.

10. Procédé selon la revendication 8, dans lequel M^{II} est Zn et M^{III} est Cr.

11. Procédé selon l'une quelconque des revendications 8 à 10, dans lequel A est choisi parmi OH, F, Cl, Br, I, SO₄, ou NO₃.

12. Procédé selon la revendication 11, dans lequel le double hydroxyde en couches est choisi parmi Mg₂Al(OH)₆NO₃2H₂O, Zn₂Cr(OH)₆NO₃2H₂O et Ni₂Al(OH)₆Cl.2H₂O.

13. Procédé selon l'une quelconque des revendications 1 à 12, grâce à quoi un nucléoside-monophosphate est séparé d'une solution le contenant et d'au moins un deuxième nucléoside-monophosphate.

14. Procédé selon la revendication 13, destiné à séparer la cytidine-5'-monophosphate de l'adénosine-5'-monophosphate.

15. Procédé selon la revendication 13, destiné à séparer la cytidine-5'-monophosphate de la guanosine-5'-monophosphate.

16. Procédé selon la revendication 13, destiné à séparer la guanosine-5'-monophosphate de l'adénosine-5'-monophosphate.

17. Procédé selon l'une quelconque des revendications 1 à 16, dans lequel le double hydroxyde en couches particulaire est mélangé avec le mélange aqueux contenant les premier et deuxième nucléosides-phosphates et en est séparé après la réaction d'intercalation.

18. Procédé selon l'une quelconque des revendications 1 à 16, dans lequel le mélange aqueux contenant le premier et le deuxième nucléosidesmonophosphates entre en contact, dans un processus continu ou discontinu, avec le double hydroxyde en couches particulaire.

19. Procédé selon l'une quelconque des revendications 1 à 18, dans lequel le nucléoside-phosphate intercalé est récupéré du double hydroxyde en couches grâce à un traitement du double hydroxyde en couches avec une solution d'un anion qui s'intercale dans le double hydroxyde en couches, de préférence au niveau du nucléoside-phosphate.

20. Procédé selon la revendication 19, dans lequel l'anion est du carbonate.

21. Procédé selon l'une quelconque des revendications 1 à 18, dans lequel le nucléoside-phosphate intercalé est récupéré du double hydroxyde en couches grâce à un traitement du double hydroxyde en couches avec un acide dans des conditions provoquant la protonation et désintercalation du nucléoside-phosphate tout en laissant les couches du double hydroxyde en couches sensiblement intactes.

22. Elément intercalé de nucléoside phosphate d'un double hydroxyde en couches ayant, avant intercalation, des couches de [M^{I}₍₁₋ₓ₎M^{III}x(OH)₂]ⁿ⁺, où M' est un cation métallique monovalent, M^{III} est un cation métallique trivalent, x est un nombre entier inférieur à 1 et n = 2x-1, le nucléoside-phosphate étant intercalé entre lesdites couches.

23. Elément intercalé selon la revendication 22, dans lequel le double hydroxyde en couches utilisé pour former l'élément intercalé a des couches de [LiAl₂(OH)₆]⁺.

24. Elément intercalé selon la revendication 22 ou 23, dans lequel le nucléoside-phosphate est un monophosphate choisi parmi la cytidine-5'-monophosphate, la guanosine-5'-monophasphate, et l'adénosine-5'-monophosphate.

25. Elément intercalé selon la revendication 22 ou 23, dans lequel le nucléoside-phosphate est de l'adénosine triphosphate.

26. Procédé de séparation d'un nucléoside phosphate d'une solution le contenant, lequel procédé comprend les étapes consistant à (1) traiter la solution avec un double hydroxyde en couches ayant la formule générale suivante :
[M^{I} ₍₁₋ₓ₎M^{III}x(OH)₂]ⁿ⁺A^{z-} _{n/z}
où M' est un cation métallique monovalent ; M^{III} est un cation métallique trivalent ; et A est un anion déplaçable, z est un nombre entier, n = 2x-1, et x est un nombre inférieur à 1, lequel double hydroxyde en couches est hydraté de façon facultative à l'aide d'une quantité d'eau stoechiométrique ou non stoechiométrique, grâce à quoi le nucléoside-phosphate est de préférence intercalé dans le double hydroxyde en couches et (2) récupérer le nucléoside-phosphate intercalé du double hydroxyde en couches.

27. Procédé selon la revendication 26, dans lequel le double hydroxyde en couches a des couches de [LiAl₂(OH)₆]⁺.

28. Procédé selon la revendication 26 ou 27, dans lequel le nucléoside-phosphate est un monophosphate choisi parmi l'adénosine-5'-monophosphate, la cytidine-5'-monophosphate et la guanosine-5'-monophosphate.

29. Procédé selon l'une quelconque des revendications 26 à 28, dans lequel le double hydroxyde en couches particulaire est mélangé avec la solution contenant le nucléoside-phosphate et en est séparé après la réaction d'intercalation.

30. Procédé selon l'une quelconque des revendications 26 à 28, dans lequel la solution contenant le nucléoside-phosphate passe à travers un corps de double hydroxyde en couches particulaire.

31. Procédé selon l'une quelconque des revendications 26 à 30, dans lequel, avant le traitement de la solution contenant le nucléoside-phosphate avec le double hydroxyde en couches, le double hydroxyde en couches est traité par irradiation ultrasonique.

32. Procédé selon l'une quelconque des revendications 26 à 31, dans lequel le nucléoside-phosphate intercalé est récupéré du double hydroxyde en couches dans lequel il est intercalé grâce au traitement du double hydroxyde en couches avec une solution d'un anion qui s'intercale dans le double hydroxyde en couches, de préférence au niveau du nucléoside-phosphate de manière à déplacer le nucléoside-phosphate du double hydroxyde en couches.

33. Procédé selon la revendication 32, dans lequel l'anion est du carbonate.

34. Procédé selon l'une quelconque des revendications 26 à 31, dans lequel le nucléoside-phosphate intercalé est récupéré du double hydroxyde en couches dans lequel il est intercalé grâce au traitement du double hydroxyde en couches avec un acide dans des conditions provoquant la protonation et la désintercalation du nucléoside-phosphate tout en laissant les couches du double hydroxyde en couches sensiblement intactes.
